Europäisches Patentamt

European Patent Office

Office européen des brevets

(11) Numéro de publication: **0 506 525 B1**

# FASCICULE DE BREVET EUROPEEN

(45) Date de publication du fascicule du brevet: **27.09.95**  (51) Int. Cl.⁶: **C07C 19/08**, C07C 17/00, B01J 23/44

(21) Numéro de dépôt: **92400743.8**

(22) Date de dépôt: **19.03.92**

Le dossier contient des informations techniques présentées postérieurement au dépôt de la demande et ne figurant pas dans le présent fascicule.

(54) **Préparation du pentafluoroéthane par hydrogénolyse du chloropentafluoroéthane.**

(30) Priorité: **27.03.91 FR 9103704**

(43) Date de publication de la demande:
**30.09.92 Bulletin 92/40**

(45) Mention de la délivrance du brevet:
**27.09.95 Bulletin 95/39**

(84) Etats contractants désignés:
**AT BE CH DE DK ES FR GB GR IT LI LU NL PT SE**

(56) Documents cités:
**EP-A- 0 349 115**
**GB-A- 2 219 796**

**CHEMICAL ABSTRACTS, vol. 112, no. 13, 26 Mars 1990, Columbus, Ohio, US; abstract no. 118521S, page 682 ;**

(73) Titulaire: **ELF ATOCHEM S.A.**
**4 & 8, Cours Michelet**
**La Défense 10**
**F-92800 Puteaux (FR)**

(72) Inventeur: **Cognion, Jean-Marie**
**85 Route de Charly**
**F-69230 Saint-Genis Laval (FR)**
Inventeur: **Guillet, Dominique**
**2 Routes de Vourlses,**
**Bâtiment B**
**F-69230 Saint-Genis Laval (FR)**

(74) Mandataire: **Leboulenger, Jean et al**
**Elf Atochem S.A.**
**Département Propriété Industrielle, Cedex 42 - La Défense 10**
**F-92091 Paris la Défense (FR)**

**Description**

La présente invention concerne le domaine des hydrocarbures fluorés et a plus particulièrement pour objet la préparation du pentafluoroéthane $CF_3$-$CF_2H$ (ci-après F125) par hydrogénation catalytique en phase vapeur du chloropentafluoroéthane $CF_3$-$CF_2Cl$ (ci-après F115).

L'hydrogénation catalytique du F115 est connue. On peut citer par exemple le rapport final de recherche de J.D. Park et J.R. Lacher publié en 1959, recherches financées par l'Air Force Office of Scientific Research (n° TR 5899) et l'Armed Services Technical Informations Agency (n° AD 162198), qui décrit l'hydrogénolyse du F115 en F125 sur un catalyseur Pd sur charbon à 250°C ; la spectrographie infra-rouge, employée comme moyen d'identification des produits de la réaction, met en évidence à côté du F125 la formation importante d'acide fluorhydrique (HF). Plus récemment, la demande de brevet japonais n° 85177/88 (publication n° 1258632) décrit pour cette réaction l'emploi de catalyseurs contenant un ou plusieurs métaux déposé(s) sur un support et choisi(s) parmi les éléments du groupe du platine, ceux du groupe du fer et le rhénium ; l'alumine est mentionnée comme support possible, mais dans tous les exemples de réalisation, le support utilisé est un charbon actif.

Des catalyseurs d'hydrogénation au palladium sur alumine, alumine fluorée ou fluorure d'aluminium ont par ailleurs (brevets EP 349115 et 379793) été utilisés pour l'hydrogénolyse du 1-chloro-1,2,2,2-tétrafluoroéthane (F124a) en 1,1,1,2-tétrafluoroéthane (F134a).

Dans l'hydrogénation catalytique du F115, la sélectivité de l'hydrogénolyse de la liaison C-Cl est très importante car l'hydrogénolyse des liaisons C-F provoque la formation d'acide fluorhydrique (FH) corrosif et de produits légers très difficilement séparables par distillation du F125 recherché :

|  | FORMULE | Eb°C/100kPa |
|---|---|---|
| F115 | $CF_3$-$CF_2Cl$ | -38,7 |
| F125 | $CF_3$-$CF_2H$ | -48,5 |
| F134a | $CF_3$-$CFH_2$ | -26,5 |
| F143a | $CF_3$-$CH_3$ | -47,5 |

Cette sélectivité n'est pas évidente à obtenir ; de nombreux brevets (voir par exemple le brevet GB 1578933 et les demandes de brevet japonais 285428/87, 288451/87, 327395/87, 67145/88 et 152278/88) citent en effet le F115 comme matière première, pouvant conduire par hydrogénolyse au F134a. Le produit le plus gênant et dont il est particulièrement difficile d'éviter la formation reste le F143a ($CF_3$-$CH_3$).

Il a maintenant été trouvé qu'il est possible d'obtenir d'excellentes sélectivités en F125 et d'éviter la formation de F143a. Le procédé selon la présente invention pour la préparation du pentafluoroéthane par hydrogénation catalytique du chloropentafluoroéthane en phase vapeur est caractérisé en ce que l'on emploie un catalyseur à base de palladium déposé sur un support d'alumine, alumine fluorée ou fluorure d'aluminium.

Dans le catalyseur utilisé selon l'invention, la teneur en palladium peut aller de 0,1 à 10 % en poids, mais est de préférence comprise entre 0,1 et 5 %.

Le support peut être une alumine, mais on préfère employer une alumine partiellement fluorée ou du fluorure d'aluminium. Par alumine partiellement fluorée, on entend un mélange d'alumine et de fluorure d'aluminium dont la teneur en $AlF_3$ est d'au moins 70 % en poids. On préfère comme supports les solides ayant une surface spécifique comprise entre 1 et 300 $m^2/g$ (de préférence entre 5 et 100 $m^2/g$), une porosité élevée (0,1 à 1 $cm^3/g$) et une granulométrie compatible avec une catalyse en lit fixe (1 à 10 mm). Ces produits sont soit commercialisés, soit préparés par fluoration d'alumine par l'acide fluorhydrique. Leur mise en forme (billes, extrudats, pastilles, produit de broyage...) peut être effectuée par les techniques habituelles avant ou après fluoration.

Le catalyseur utilisé selon l'invention peut être préparé par les techniques classiques d'imprégnation du support au moyen d'une solution aqueuse ou organique d'un dérivé du palladium. Il est connu que la distribution de la phase active catalytique sur un support peut être contrôlée par la méthode d'imprégnation, par la nature du précurseur métallique et par la nature, la quantité et le pH de la solution employée. Il est ainsi possible d'obtenir une pénétration plus ou moins importante de la phase active dans le support. Une imprégnation limitée à l'extérieur des grains du support, connue dans le métier comme imprégnation en "coquille d'oeuf", peut être dans certains cas favorable à l'activité et à la sélectivité de la réaction.

Après imprégnation, l'eau ou le solvant organique est éliminé par évaporation et le solide obtenu est soumis à un traitement thermique à une température allant de 100 à 500°C (de préférence 200 à 350°C) et sous courant d'hydrogène et/ou d'azote (de préférence sous une pression de 1 à 5 bars) pour libérer le palladium. La nature du dérivé du palladium est sans importance et peut être par exemple le chlorure, l'acétate, l'acétylacétonate, le nitrate ou les complexes organométalliques du chlorure. Le solvant peut être l'eau ou un composé organique. Comme solvants organiques on peut utiliser les dérivés chlorés du méthane ou de l'éthane (par exemple le chloroforme, le chlorure de méthylène et le tétrachlorure de carbone), les solvants aromatiques (par exemple le benzène, le toluène et le chlorobenzène) ou des amines ou alcanolamines (par exemple la pyridine et l'éthanolamine).

L'hydrogénation catalytique du F115 selon l'invention peut être effectuée à une température allant de 100 à 400°C (de préférence entre 200 et 350°C), avec un rapport molaire hydrogène/F115 allant de 0,5 à 4 (de préférence 1 à 2), sous une pression de 1 à 50 bars et un débit horaire de 0,5 à 12 moles de F115 par litre de catalyseur.

Les exemples suivants illustrent l'invention sans la limiter. Les résultats sont exprimés par le taux de transformation ($TT_G$) du F115 et la sélectivité (S) en un produit de la réaction :

$$TT_G = 100 \times \frac{\text{Nombre de moles de F115 transformé}}{\text{Nombre de moles de F115 introduit}}$$

$$S = 100 \times \frac{\text{Nombre de moles de produit formé}}{\text{Nombre de moles de F115 transformé}}$$

et sont obtenus par des analyses en ligne entrée et sortie réacteur (après lavage à l'eau) associées aux mesures des débits correspondants. Les principaux sous-produits de la réaction sont le F134a ($CF_3$-$CFH_2$) valorisable et le F143a ($CF_3$-$CH_3$) dont la limite détectable par l'analyse en ligne sortie réacteur est de 0,05 %.

## EXEMPLE 1

### a) Préparation du catalyseur

Dans un évaporateur rotatif, on charge 50 ml (28 g) d'une alumine fluorée contenant 77 % en poids d'$AlF_3$ et ayant une porosité de 0,77 $cm^3/g$ et une surface spécifique de 67 $m^2/g$, sous forme de billes de 1,5 à 2 mm de diamètre. Après dégazage pendant 3 heures à 100°C sous pression réduite (1 kPa), on introduit 50 ml d'une solution d'acétate de palladium dans le toluène contenant 0,28 g de Pd, puis on évapore le solvant sous pression réduite (26 kPa) et sèche à 80°C. On traite ensuite à 250°C pendant 2 heures sous un courant d'azote (5 Nl/h) et obtient ainsi un catalyseur contenant 1 % Pd déposé en "coquille d'oeuf" (catalyseur A).

### b) Hydrogénation du F115

Dans un tube en Inconel de 45 cm de long et de 2,72 cm de diamètre intérieur, chauffé électriquement, on introduit 50 ml du catalyseur A obtenu précédemment, puis on y fait passer un mélange d'hydrogène et de F115 à la pression atmosphérique, aux rapports molaires, débits et températures indiqués dans le tableau 1 suivant dont la dernière partie rassemble les résultats obtenus. L'analyse en ligne sortie réacteur ne détecte pas la présence de F143a.

TABLEAU 1

| ESSAI N° | 11 | 12 | 13 | 14 |
|---|---|---|---|---|
| Conditions opératoires : | | | | |
| Température ($^\circ$C) <br> Rapport molaire $H_2/C_2F_5Cl$ <br> Débit $C_2F_5Cl$ (mole/heure) | 250 <br> 2 <br> 0,09 | 300 <br> 2 <br> 0,09 | 300 <br> 2 <br> 0,045 | 300 <br> 2 <br> 0,025 |
| Résultats : | | | | |
| % $TT_G$ du $C_2F_5Cl$ <br> % S en $C_2F_5H$ | 14 <br> 100 | 45 <br> 91 | 57 <br> 99 | 70 <br> 97,5 |

## EXEMPLE 2

### a) Préparation du catalyseur

Dans un évaporateur rotatif, on charge 50 ml (36 g) d'une alumine fluorée contenant 97 % en poids d'$AlF_3$ et ayant une porosité de 0,42 cm$^3$/g et une surface spécifique de 110 m$^2$/g sous forme de pastilles de 5 mm de diamètre et de 3 mm d'épaisseur. Après dégazage pendant 3 heures à 100 $^\circ$C sous pression réduite (1 kPa), on introduit 50 ml d'une solution d'acétate de palladium dans le toluène contenant 1,9 g de Pd, puis on évapore le solvant sous pression réduite (26 kPa) et sèche à 80 $^\circ$C. On traite ensuite à 250 $^\circ$C pendant 2 heures sous un courant d'azote (5 Nl/h) et obtient ainsi un catalyseur contenant 5 % de Pd déposé en "coquille d'oeuf" (catalyseur B).

### b) Hydrogénation du F115

Dans le même appareillage qu'à l'exemple 1-b, on introduit 50 ml d'une charge de catalyseur B sur laquelle on effectue successivement différents essais d'hydrogénation du chloropentafluoroéthane à la pression atmosphérique.

Les conditions opératoires des essais et les résultats obtenus sont rassemblés dans le tableau 2 suivant. L'analyse en ligne sortie réacteur ne détecte pas la présence de F143a.

TABLEAU 2

| ESSAI N° | 21 | 22 | 23 | 24 | 25 |
|---|---|---|---|---|---|
| Conditions opératoires : | | | | | |
| Température ($^\circ$C) <br> Rapport molaire $H_2/C_2F_5Cl$ <br> Débit $C_2F_5Cl$ (mole/heure) | 250 <br> 2 <br> 0,09 | 250 <br> 2 <br> 0,05 | 250 <br> 2 <br> 0,02 | 250 <br> 3,8 <br> 0,030 | 250 <br> 1,1 <br> 0,065 |
| Résultats : | | | | | |
| % $TT_G$ du $C_2F_5Cl$ <br> % S en $C_2F_5H$ | 70 <br> 99 | 82 <br> 97,5 | 91,5 <br> 97 | 88,5 <br> 99 | 82 <br> 97,5 |

4

## EXEMPLE 3

### a) Préparation du catalyseur

Dans un évaporateur rotatif, on charge 50 ml (28 g) d'une alumine fluorée contenant 82 % en poids d'AlF$_3$ et ayant une porosité de 0,74 cm$^3$/g et une surface spécifique de 67 m$^2$/g sous forme de billes de 1,5 à 2 mm de diamètre. Après dégazage pendant 3 heures à 100°C sous pression réduite (1 kPa), on introduit 25 ml d'une solution d'acétate de palladium dans le chlorure de méthylène contenant 1,5 g de Pd, puis on évapore le solvant sous pression réduite (26 kPa) et sèche à 80°C. On traite ensuite à 300°C pendant 2 heures sous un courant d'hydrogène (5 Nl/h) et obtient ainsi un catalyseur contenant 5 % de Pd déposé uniformément (catalyseur C).

### b) Hydrogénation du F115

Dans le même appareillage qu'à l'exemple 1-b, on introduit 50 ml d'une charge de catalyseur C sur laquelle on effectue successivement différents essais d'hydrogénation du chloropentafluoroéthane à la pression atmosphérique.

Les conditions opératoires des essais et les résultats obtenus sont rassemblés dans le tableau 3 suivant. L'analyse en ligne sortie réacteur ne détecte pas la présence de F143a.

TABLEAU 3

| ESSAI N° | 31 | 32 | 33 |
|---|---|---|---|
| **Conditions opératoires :** | | | |
| Température (°C) | 250 | 250 | 250 |
| Rapport molaire H$_2$/C$_2$F$_5$Cl | 2 | 1,9 | 1,8 |
| Débit C$_2$F$_5$Cl (mole/heure) | 0,09 | 0,05 | 0,025 |
| **Résultats :** | | | |
| % TT$_G$ du C$_2$F$_5$Cl | 32,5 | 47 | 59,6 |
| % S en C$_2$F$_5$H | 99,5 | 99,5 | 98 |

## EXEMPLE 4

### a) Préparation du catalyseur

Dans un évaporateur rotatif, on charge 50 ml (28 g) d'une alumine fluorée contenant 77 % en poids d'AlF$_3$ et ayant une porosité de 0,72 cm$^3$/g et une surface spécifique de 67 m$^2$/g sous forme de billes de 1,5 à 2 mm de diamètre. Après dégazage pendant 3 heures à 100°C sous pression réduite (1 kPa), on introduit 50 ml d'une solution aqueuse de chlorure de palladium contenant 1,45 g de Pd, puis on évapore l'eau sous pression réduite (1 kPa) et sèche à 100°C. On traite ensuite à 400°C pendant 2 heures sous un courant d'hydrogène (5 Nl/h) et obtient ainsi un catalyseur contenant 5 % de Pd imprégné uniformément (catalyseur D).

### b) Hydrogénation du F115

Dans le même appareillage qu'à l'exemple 1-b, on introduit une charge de 50 ml de catalyseur D sur laquelle on effectue successivement différents essais d'hydrogénation du chloropentafluoroéthane (essais 41 à 44). On procède de même avec une charge de 10 ml de catalyseur D (essais 41* à 46*).

Les conditions opératoires des essais et les résultats obtenus sont rassemblés dans le tableau 4 suivant. L'analyse en ligne sortie réacteur ne détecte pas la présence de F143a.

**TABLEAU 4**

| ESSAI N° | 41 | 42 | 43 | 44 | 41* | 42* | 43* | 44* | 45* | 46* |
|---|---|---|---|---|---|---|---|---|---|---|
| **Conditions opératoires :** | | | | | | | | | | |
| Catalyseur (cm$^3$) | 50 | 50 | 50 | 50 | 10 | 10 | 10 | 10 | 10 | 10 |
| Température (°C) | 250 | 250 | 250 | 300 | 250 | 250 | 250 | 180 | 250 | 250 |
| Pression (bar) | 1 | 1 | 1 | 1 | 10 | 10 | 10 | 30 | 30 | 30 |
| Rapport molaire H$_2$/C$_2$F$_5$Cl | 2 | 2 | 1,8 | 1,8 | 2 | 1,9 | 1,8 | 2 | 1,8 | 2,1 |
| Débit C$_2$F$_5$Cl (mole/heure) | 0,09 | 0,05 | 0,025 | 0,025 | 0,095 | 0,05 | 0,025 | 0,09 | 0,05 | 0,025 |
| **Résultats :** | | | | | | | | | | |
| % TT$_G$ du C$_2$F$_5$Cl | 70,5 | 82 | 91,5 | 100 | 21,5 | 41 | 44 | 23 | 30,5 | 39 |
| % S en C$_2$F$_5$H | 98,5 | 98 | 98 | 97 | 99 | 99 | 98,5 | 99 | 96 | 94 |

6

## EXEMPLE 5

### a) Préparation des catalyseurs

Dans un évaporateur rotatif, on charge 50 ml (28 g) d'une alumine fluorée contenant 82 % en poids d'AlF$_3$ et ayant une porosité de 0,74 cm$^3$/g et une surface spécifique de 62 m$^2$/g sous forme de billes de 1,5 à 2 mm de diamètre. Après dégazage pendant 3 heures à 100°C sous pression réduite (1 kPa), on introduit 50 ml d'une solution aqueuse de chlorure de palladium contenant 0,14 g de Pd, puis on évapore l'eau sous pression réduite (1 kPa) et sèche à 100°C. On traite ensuite à 360°C pendant 2 heures sous un courant d'hydrogène (5 Nl/h) et obtient ainsi un catalyseur contenant 0,5 % de Pd imprégné uniformément (catalyseur E).

Dans un évaporateur rotatif, on charge 50 ml (28 g) d'une alumine fluorée contenant 82 % en poids d'AlF$_3$ et ayant une porosité de 0,74 cm$^3$/g et une surface spécifique de 67 m$^2$/g sous forme de billes de 1,5 à 2 mm de diamètre. Après dégazage pendant 3 heures à 100°C sous pression réduite (1 kPa), on introduit 50 ml d'une solution d'acétate de palladium dans le toluène contenant 0,14 g de Pd, puis on évapore le solvant sous pression réduite (26 kPa) et sèche à 80°C. On traite ensuite à 360°C pendant 2 heures sous un courant d'hydrogène (5 Nl/h) et obtient ainsi un catalyseur contenant 0,5 % de Pd déposé en "coquille d'oeuf" (catalyseur F).

### b) Hydrogénation du F115

Dans le même appareillage qu'à l'exemple 1-b, on introduit une charge de 50 ml du catalyseur E ou F à 0,5 % de Pd sur lequel on effectue différents essais d'hydrogénation du chloropentafluoroéthane à pression atmosphérique.

Les conditions opératoires des essais et les résultats obtenus sont rassemblés dans le tableau 5 suivant. L'analyse en ligne sortie réacteur ne détecte pas la présence de F143a. Le catalyseur imprégné en "coquille d'oeuf" (catalyseur F) s'avère supérieur au catalyseur imprégné uniformément (catalyseur E).

TABLEAU 5

| ESSAI N° | 51 | 52 | 53 | 54 |
|---|---|---|---|---|
| Conditions opératoires : | | | | |
| Catalyseur | E | E | F | F |
| Température (°C) | 250 | 250 | 250 | 250 |
| Rapport molaire H$_2$/C$_2$F$_5$Cl | 1,9 | 1,8 | 1,9 | 1,8 |
| Débit C$_2$F$_5$Cl (mole/heure) | 0,045 | 0,025 | 0,046 | 0,025 |
| Résultats : | | | | |
| % TT$_G$ du C$_2$F$_5$Cl | 15,5 | 21 | 19 | 29 |
| % S en C$_2$F$_5$H | 97 | 97 | 99 | 97 |

## EXEMPLE 6

### a) Préparation du catalyseur

Dans un évaporateur rotatif, on charge 50 ml (20 g) d'une alumine ayant une porosité de 1 cm$^3$/g et une surface spécifique de 280 m$^2$/g sous forme de billes de 1,4 à 1,7 mm de diamètre. Après dégazage pendant 3 heures à 100°C sous pression réduite (1 kPa), on introduit 50 ml d'une solution d'acétate de palladium dans le toluène contenant 0,2 g de Pd, puis on évapore le solvant sous pression réduite (26 kPa) et sèche à 80°C. On traite ensuite à 250°C pendant 2 heures sous un courant d'azote (3 Nl/h) et obtient ainsi un catalyseur contenant 1 % de Pd déposé en "coquille d'oeuf" (catalyseur G).

### b) Hydrogénation du F115

Dans le même appareillage qu'à l'exemple 1-b, on introduit 50 ml d'une charge de catalyseur G sur laquelle on effectue l'hydrogénation du chloropentafluoroéthane dans les conditions suivantes :
- pression atmosphérique
- température : 300°C
- rapport molaire $H_2/C_2F_5Cl$ : 1,9
- débit $C_2F_5Cl$ : 0,045 mole/heure

Le TTG du $C_2F_5Cl$ est de 49 % et la sélectivité en $C_2F_5H$ atteint 92 %. L'analyse en ligne sortie réacteur ne détecte pas la présence de F143a.

## EXEMPLE 7

### a) Préparation du catalyseur

Dans un évaporateur rotatif, on charge 50 ml (25 g) d'une alumine fluorée contenant 82 % en poids d'$AlF_3$ et ayant une porosité de 0,74 cm$^3$/g et une surface spécifique de 67 m$^2$/g sous forme de billes de 1,5 à 2 mm de diamètre. Après dégazage pendant 3 heures à 100°C sous pression réduite (1 kPa), on introduit 50 ml d'une solution aqueuse de chlorure de palladium contenant 0,25 g de Pd, puis on évapore l'eau sous pression réduite (1 kPa) et sèche à 100°C. On traite ensuite à 300°C pendant 2 heures sous un courant d'hydrogène (5 Nl/h) et obtient ainsi un catalyseur contenant 1 % de Pd déposé uniformément (catalyseur H).

### b) Hydrogénation du F115

Dans le même appareillage qu'à l'exemple 1-b, on introduit une charge de 50 ml de catalyseur H sur laquelle on effectue différents essais d'hydrogénation du chloropentafluoroéthane à la pression atmosphérique.

Les conditions opératoires des essais et les résultats obtenus sont rassemblés dans le tableau 6 suivant. L'analyse en ligne sortie réacteur ne détecte pas la présence de F143a.

TABLEAU 6

| ESSAI N° | 71 | 72 | 73 |
|---|---|---|---|
| **Conditions opératoires :** | | | |
| Température (°C) | 300 | 300 | 300 |
| Rapport molaire $H_2/C_2F_5Cl$ | 2 | 1,9 | 1,8 |
| Débit $C_2F_5Cl$ (mole/heure) | 0,09 | 0,05 | 0,025 |
| **Résultats :** | | | |
| % $TT_G$ du $C_2F_5Cl$ | 17,5 | 34,8 | 43,3 |
| % S en $C_2F_5H$ | 98,5 | 98 | 95 |

## EXEMPLE 8 (Comparatif)

### a) Préparation d'un catalyseur Pd/C non conforme à l'invention

Dans un évaporateur rotatif, on charge 60 ml (28 g) d'un charbon actif ayant une porosité de 0,6 cm$^3$/g et une surface spécifique de 950 m$^2$/g sous forme d'extrudés de 1,8 mm de diamètre. Après dégazage pendant 3 heures à 100°C sous pression réduite (1 kPa), on introduit 70 ml d'une solution aqueuse de chlorure de palladium contenant 1,5 g de Pd, puis on évapore l'eau sous pression réduite (1 kPa) et sèche à 100°C. On traite ensuite à 400°C pendant 2 heures sous un courant d'hydrogène (10 Nl/h) et obtient ainsi un catalyseur contenant 5 % de Pd sur charbon actif.

### b) Hydrogénation du F115

Dans le même appareillage qu'à l'exemple 1-b, on introduit 25 ml du catalyseur Pd/C préparé ci-dessus sur lequel on effectue différents essais d'hydrogénation du chloropentafluoroéthane à la pression atmosphérique.

Les conditions opératoires des essais et les résultats obtenus sont rassemblés dans le tableau 7 suivant. On remarque la formation importante de F143a.

TABLEAU 7

| ESSAI N° | 81 | 82 | 83 | 84 |
|---|---|---|---|---|
| **Conditions opératoires :** | | | | |
| Température (°C) | 200 | 250 | 300 | 350 |
| Rapport molaire $H_2/C_2F_5Cl$ | 1,9 | 1,9 | 1,9 | 1,9 |
| Débit $C_2F_5Cl$ (mole/heure) | 0,045 | 0,045 | 0,045 | 0,045 |
| **Résultats :** | | | | |
| % $TT_G$ du $C_2F_5Cl$ | 2 | 40 | 91,5 | 100 |
| % S en $C_2F_5H$ | 99,5 | 99 | 89,5 | 83 |
| % S en $CF_3CH_3$ | < 0,4 | 1 | 3,8 | 11,5 |

## EXEMPLE 9 (Comparatif)

### a) Préparation d'un catalyseur Pt/alumine fluorée, non conforme à l'invention

Dans un évaporateur rotatif, on charge 50 ml (28 g) d'une alumine fluorée contenant 82 % en poids d'$AlF_3$ et ayant une porosité de 0,74 cm$^3$/g et une surface spécifique de 67 m$^2$/g sous forme de billes de 1,5 à 2 mm de diamètre. Après dégazage pendant 3 heures à 100°C sous pression réduite (1 kPa), on introduit 50 ml d'une solution aqueuse d'acide hexachloroplatinique contenant 0,3 g de Pt, puis on évapore l'eau sous pression réduite (1 kPa) et sèche à 100°C. On traite ensuite à 400°C pendant 2 heures sous un courant d'hydrogène (5 Nl/h) et obtient ainsi un catalyseur contenant 1 % de Pt déposé uniformément sur le support.

### b) Hydrogénation du F115

Dans le même appareillage qu'à l'exemple 1-b, on introduit 50 ml de ce catalyseur au platine sur lequel on effectue différents essais d'hydrogénation du chloropentafluoroéthane à la pression atmosphérique.

Les conditions opératoires des essais et les résultats obtenus sont rassemblés dans le tableau 8 suivant. L'analyse en ligne sortie réacteur ne met pas en évidence la formation de F143a, mais l'activité du catalyseur est très faible.

TABLEAU 8

| ESSAI N° | 91 | 92 |
|---|---|---|
| Conditions opératoires : | | |
| Température (°C)<br>Rapport molaire $H_2/C_2F_5Cl$<br>Débit $C_2F_5Cl$ (mole/heure) | 300<br>1,8<br>0,025 | 350<br>1,8<br>0,025 |
| Résultats : | | |
| % $TT_G$ du $C_2F_5Cl$<br>% S en $C_2F_5H$ | 11<br>71 | 29<br>45 |

## EXEMPLE 10

### a) Préparation du catalyseur

Dans un évaporateur rotatif, on charge 50 ml (39 g) d'une alumine fluorée contenant 84 % en poids d'$AlF_3$ et ayant une porosité de 0,4 $cm^3/g$ et une surface spécifique de 12 $m^2/g$ sous forme de billes de 1,5 à 1,6 mm de diamètre. Après dégazage pendant 3 heures à 100°C sous pression réduite (1 kPa), on introduit 20 ml d'une solution aqueuse de nitrate de palladium contenant 0,195 g de Pd, puis on évapore l'eau sous pression réduite (1 kPa) et sèche à 100°C. On traite ensuite à 250°C pendant 2 heures sous un courant d'azote (2 Nl/h) et obtient ainsi un catalyseur contenant 0,5 % de Pd déposé en "coquille d'oeuf" (catalyseur I).

### b) Hydrogénation du F115

Dans le même appareillage qu'à l'exemple 1-b, on introduit une charge de 50 ml de catalyseur I sur laquelle on effectue successivement différents essais d'hydrogénation du chloropentafluoroéthane à la pression atmosphérique.

Les conditions opératoires des essais et les résultats obtenus sont rassemblés dans le tableau 9 suivant. L'analyse en ligne sortie réacteur ne détecte pas la présence de F143a.

TABLEAU 9

| ESSAI N° | 101 | 102 | 103 |
|---|---|---|---|
| Conditions opératoires : | | | |
| Température (°C)<br>Rapport molaire $H_2/C_2F_5Cl$<br>Débit $C_2F_5Cl$ (mole/heure) | 250<br>2<br>0,046 | 250<br>2<br>0,023 | 250<br>2<br>0,09 |
| Résultats : | | | |
| % $TT_G$ du $C_2F_5Cl$<br>% S en $C_2F_5H$ | 27<br>96 | 38<br>96 | 22<br>97 |

## EXEMPLE 11

### a) Préparation du catalyseur

Dans un évaporateur rotatif, on charge 50 ml (51 g) de fluorure d'aluminium $AlF_3$, ayant une porosité de 0,25 $cm^3$/g et une surface spécifique de 10 $m^2$/g sous forme de billes de 1,8 à 2 mm de diamètre. Après dégazage pendant 3 heures à 100°C sous pression réduite (1 kPa), on introduit 20 ml d'une solution aqueuse de nitrate de palladium contenant 0,25 g de Pd, puis on évapore l'eau sous pression réduite (1 kPa) et sèche à 100°C. On traite ensuite à 250°C pendant 2 heures sous un courant d'azote (2 Nl/h) et obtient ainsi un catalyseur contenant 0,5 % de Pd déposé en "coquille d'oeuf" (catalyseur J).

### b) Hydrogénation du F115

Dans le même appareillage qu'à l'exemple 1-b, on introduit une charge de 50 ml de catalyseur J sur laquelle on effectue successivement différents essais d'hydrogénation du chloropentafluoroéthane à la pression atmosphérique.

Les conditions opératoires des essais et les résultats obtenus sont rassemblés dans le tableau suivant. L'analyse en ligne sortie réacteur ne détecte pas la présence de F143a.

TABLEAU 10

| ESSAI N° | 111 | 112 | 113 |
|---|---|---|---|
| Conditions opératoires : | | | |
| Température (°C) | 300 | 300 | 300 |
| Rapport molaire $H_2/C_2F_5Cl$ | 2 | 2 | 2 |
| Débit $C_2F_5Cl$ (mole/heure) | 0,023 | 0,089 | 0,046 |
| Résultats : | | | |
| % $TT_G$ du $C_2F_5Cl$ | 97,4 | 75,3 | 84 |
| % S en $C_2F_5H$ | 99,3 | 95,8 | 97,2 |

## EXEMPLE 12

### a) Préparation du catalyseur

Dans un évaporateur rotatif, on charge 50 ml (30 g) d'une alumine fluorée contenant 75 % en poids d'$AlF_3$ et ayant une porosité de 0,72 $cm^3$/g et une surface spécifique de 71 $m^2$/g sous forme de billes de 1,5 à 2 mm de diamètre. Après dégazage pendant 3 heures à 100°C sous pression réduite (1 kPa), on introduit 23 ml d'une solution aqueuse de nitrate de palladium contenant 0,30 g de Pd, puis on évapore l'eau sous pression réduite (1 kPa) et sèche à 100°C. On traite ensuite à 250°C pendant 2 heures sous un courant d'azote (2 Nl/h) et obtient ainsi un catalyseur contenant 1 % de Pd déposé "en coquille d'oeuf" (catalyseur K).

### b) Hydrogénation du F115

Dans le même appareillage qu'à l'exemple 1-b, on introduit une charge de 50 ml de catalyseur K sur laquelle on effectue successivement différents essais d'hydrogénation du chloropentafluoroéthane à la pression atmosphérique.

Les conditions opératoires des essais et les résultats obtenus sont rassemblés dans le tableau suivant. L'analyse en ligne sortie réacteur ne détecte pas la présence de F143a.

TABLEAU 11

| ESSAI N° | 121 | 122 |
|---|---|---|
| Conditions opératoires : | | |
| Température ($°C$) | 250 | 300 |
| Rapport molaire $H_2/C_2F_5Cl$ | 1,9 | 1,1 |
| Débit $C_2F_5Cl$ (mole/heure) | 0,024 | 0,124 |
| Résultats : | | |
| % $TT_G$ du $C_2F_5Cl$ | 42 | 51 |
| % S en $C_2F_5H$ | 98 | 93 |

**Revendications**

1. Procédé de préparation du pentafluoroéthane par hydrogénation catalytique en phase vapeur du chloropentafluoroéthane, caractérisé en ce que l'on emploie un catalyseur à base de palladium déposé sur un support d'alumine, alumine fluorée ou fluorure d'aluminium.

2. Procédé selon la revendication 1, dans lequel la teneur en palladium du catalyseur est comprise entre 0,1 et 10 % en poids, de préférence entre 0,1 et 5 %.

3. Procédé selon la revendication 1 ou 2, dans lequel la surface spécifique du support est comprise entre 1 et 300 $m^2/g$, de préférence entre 5 et 100 $m^2/g$, sa porosité est comprise entre 0,1 et 1 $cm^3/g$, et sa granulométrie entre 1 et 10 mm.

4. Procédé selon l'une des revendications 1 à 3, dans lequel le catalyseur est préparé par imprégnation du support au moyen d'une solution aqueuse ou organique d'un dérivé du palladium, puis élimination de l'eau ou du solvant organique par évaporation et enfin traitement thermique à une température allant de 100 à 500°C sous un courant d'hydrogène et/ou d'azote.

5. Procédé selon la revendication 4, dans lequel l'imprégnation est limitée à l'extérieur des grains du support.

6. Procédé selon l'une des revendications 1 à 5, dans lequel l'hydrogénation du chloropentafluoroéthane est effectuée à une température comprise entre 100 et 400°C, de préférence entre 200 et 350°C.

7. Procédé selon la revendication 6, dans lequel l'hydrogénation du chloropentafluoroéthane est effectuée sous une pression allant de 1 à 50 bars.

8. Procédé selon la revendication 6 ou 7, dans lequel le rapport molaire : hydrogène/chloropentafluoroéthane est compris entre 0,5 et 4, de préférence entre 1 et 2.

9. Procédé selon l'une' des revendications 6 à 8, dans lequel le débit horaire de chloropentafluoroéthane est compris entre 0,5 et 12 moles par litre de catalyseur.

**Claims**

1. Process for the preparation of pentafluoroethane by vapour phase catalytic hydrogenation of chloropentafluoroethane, characterized in that a catalyst based on palladium deposited on an alumina, fluorinated alumina or aluminium fluoride support is employed.

2. Process according to Claim 1, in which the palladium content of the catalyst is between 0.1 and 10 % by weight, preferably between 0.1 and 5 %.

12

3. Process according to Claim 1 or 2, in which the specific surface area of the support is between 1 and 300 m$^2$/g, preferably between 5 and 100 m$^2$/g, its porosity is between 0.1 and 1 cm$^3$/g, and its particle size range between 1 and 10 mm.

4. Process according to one of Claims 1 to 3, in which the catalyst is prepared by impregnation of the support by means of an aqueous or organic solution of a palladium derivative, followed by removal of the water or of the organic solvent by evaporation and finally heat treatment at a temperature ranging from 100 to 500°C under a stream of hydrogen and/or nitrogen.

5. Process according to Claim 4, in which the impregnation is limited to the exterior of the support particles.

6. Process according to one of Claims 1 to 5, in which the hydrogenation of chloropentafluoroethane is performed at a temperature of between 100 and 400°C, preferably between 200 and 350°C.

7. Process according to Claim 6, in which the hydrogenation of chloropentafluoroethane is performed at a pressure ranging from 1 to 50 bars.

8. Process according to Claim 6 or 7, in which the hydrogen/chloropentafluoroethane molar ratio is between 0.5 and 4, preferably between 1 and 2.

9. Process according to one of Claims 6 to 8, in which the hourly flow rate of chloropentafluoroethane is between 0.5 and 12 moles per litre of catalyst.

**Patentansprüche**

1. Verfahren zur Herstellung von Pentafluorethan durch katalytische Hydrogenolyse von Chlorpentafluorethan in gasförmiger Phase, dadurch gekennzeichnet, daß man einen Katalysator auf Basis von Palladium verwendet, der auf einen Träger aus Aluminiumoxid, fluoriertem Aluminiumoxid oder Aluminiumfluorid aufgebracht ist.

2. Verfahren nach Anspruch 1, wobei der Palladiumgehalt des Katalysators zwischen 0,1 und 10 Gew.-%, vorzugsweise zwischen 0,1 und 5 Gew.-%, liegt.

3. Verfahren nach Anspruch 1 oder 2, wobei die spezifische Oberfläche des Trägermaterials zwischen 1 und 300 m$^2$/g, vorzugsweise zwischen 5 und 100 m$^2$/g, seine Porosität zwischen 0,1 und 1 cm$^3$/g und seine Granulometrie zwischen 1 und 10 mm liegt.

4. Verfahren nach einem der Ansprüche 1 bis 3, wobei der Katalysator durch Imprägnieren des Trägermaterials mittels einer wäßrigen oder organischen Lösung einer Palladiumverbindung hergestellt wird, gefolgt von einer Entfernung des Wassers oder des organischen Lösungsmittels durch Verdampfung und anschließender thermischer Behandlung bei einer Temperatur zwischen 100 und 500 °C unter einem Wasserstoff- und/oder Stickstoffstrom.

5. Verfahren nach Anspruch 4, wobei die Imprägnierung auf die Außenseite der Kugeln des Trägermaterials begrenzt ist.

6. Verfahren nach einem der Ansprüche 1 bis 5, wobei die Hydrogenolyse von Chlorpentafluorethan bei einer Temperatur zwischen 100 und 400 °C, vorzugsweise zwischen 200 und 350 °C, durchgeführt wird.

7. Verfahren nach Anspruch 6, wobei die Hydrogenolyse von Chlorpentafluorethan unter einem Druck zwischen 1 und 50 bar durchgeführt wird.

8. Verfahren nach Anspruch 6 bis 7, wobei das Wasserstoff/Chlorpentafluorethan-Molverhältnis zwischen 0,5 und 4, vorzugsweise zwischen 1 und 2, liegt.

9.  Verfahren nach einem der Ansprüche 6 bis 8, wobei der stündliche Durchsatz von Chlorpentafluorethan zwischen 0,5 und 12 Mol pro Liter Katalysator beträgt.